(19) Europäisches Patentamt
European Patent Office
Office européen des brevets

(11) **EP 0 970 706 A1**

(12) **EUROPÄISCHE PATENTANMELDUNG**

(43) Veröffentlichungstag:
**12.01.2000 Patentblatt 2000/02**

(51) Int. Cl.⁷: **A61L 9/00**, A61L 9/04

(21) Anmeldenummer: **99105622.7**

(22) Anmeldetag: **19.03.1999**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **07.07.1998 DE 19830348**

(71) Anmelder: **Hager, Herbert**
**66809 Nalbach (DE)**

(72) Erfinder: **Hager, Herbert**
**66809 Nalbach (DE)**

(74) Vertreter: **Klein, Friedrich**
**Auf der Pirsch 11**
**67663 Kaiserslautern (DE)**

(54) **Verfahren zum Desinfizieren von Luft**

(57)    Die Erfindung betrifft ein Verfahren zur Desinfektion von Luft, wobei die zu desinfizierende Luft mit Wasserstoffperoxid angereichert wird, um durch katalytische Zersetzung des Wasserstoffperoxid-Luftgemisches HO-Radikale aus dem Wasserstoffperoxid auszulösen, die innerhalb des Katalysators auf das Wasserstoffperoxid-Luftgemisch einwirken, sodaß diese einerseits ihre bakterizide Wirkung auf den Luftstrom ausüben und sie andererseits unter Bildung neuer HO-Radikale in Form von Kettenreaktionen wiederholt mit dem Wasserstoffperoxid-Luftgemisch reagieren;

EP 0 970 706 A1

Printed by Xerox (UK) Business Services
2.16.7/3.6

**Beschreibung**

Die Erfindung betrifft ein Verfahren zur Desinfektion von Luft.

Zum Reinigen oder zur Desinfektion von Luft sind die verschiedensten Verfahren bekannt. Diese beruhen meist darauf, daß die zu reinigende bzw zu desinfizierende Luft mit einem entsprechenden Medium in Verbindung gebracht wird, um dadurch der Luft ihre Schmutz- und/oder Bakterienanteile zu entziehen, oder letztere abzutöten.

Dabei ist es bekannt, daß Ozon, atomarer Sauerstoff und HO-Radikale zu den stärksten Oxidationsmitteln gehören und aufgrund ihrer starken Oxidationskraft eine sehr starke bakterizide Wirkung ausüben.

Der Erfindung liegt die Aufgabe zugrunde, ein Verfahren zur Desinfektion von Luft zu schaffen, das einerseits hinsichtlich des Aufwandes sowohl der erforderlichen Verbrauchsstoffe, als auch der zu seiner Durchführung erforderlichen Apparatur kostengünstig ist und das andererseits keine gesundheitsschädlichen Endstoffe erzeugt.

Diese Aufgabe wird erfindungsgemäß dadurch gelöst, daß der zu desinfizierende Luftstrom mit Wasserstoffperoxid angereichert wird, und das Wasserstoffperoxid bzw das Wasserstoffperoxid-Luftgemisch katalytisch zersetzt wird, um aus dem Wasserstoffperoxid HO-Radikale auszulösen, die innerhalb des Katalysators auf das Wasserstoffperoxid-Luftgemisch einwirken, um einerseits ihre bakterizide Wirkung auf den Luftstrom auszuüben und andererseits unter Bildung neuer HO-Radikale in Form von Kettenreaktionen wiederholt mit dem Wasserstoffperoxid-Luftgemisch im Sinn eines Herauslösens neuer HO-Radikale zu reagieren;

Wasserstoffperoxid ist bekannt und zeigt bei Zimmertemperatur eine nahezu unmessbare Zerfallgeschwindigkeit, sodaß Wasserstoffperoxid praktisch als beständig anzusehen ist. Die große Zerfallshemmung von Wasserstoffperoxid beruht dabei im wesentlichen darauf, daß der erste Schrift der Wasserstoffperoxid Thermolyse in einer enrgieaufwendigen Molekülspaltung in zwei HO-Radikale besteht.

Läßt man auf Wasserstoffperoxid Wärmeenergie einwirken, so lassen sich HO-Radikale nach der Gleichung

$$211 \text{ KJ} + HOOH \rightarrow 2 \text{ HO}$$

freisetzen.

Diese HO-Radikale lösen eine Kettenreaktion entsprechend den Gleichungen

$$HO + H_2O_2 \rightarrow H_2O + HO_2$$

$$HO_2 + H_2O_2 \rightarrow H_2O + O_2 + HO$$

aus, wodurch erneut HO-Radikale entstehen.

Durch die Anwendung eines Katalysators, vorteilhafter Weise eines mit Platinmetall oder mit Metalloxid bestückten Katalysators, oder eines als Wirkstoff Stoffe mit rauher Oberfläche aufweisenden Katalysators läßt sich die Zersetzungsgeschwindigkeit sehr stark erhöhen, sodaß der Zerfallprozess schon bei Raumtemperatur ablaufen kann.

Mit dem erfindungsgemäßen Verfahren wird unter Ausnutzung der Beschleunigung des Zerfallprozesses von Wasserstoffperoxid durch einen Katalysator ein mit diesem angereicherter Luftstrom in einen Katalysator geführt. Im Wirkbereich des Katalysators zerfällt das Wasserstoffperoxid unter Bildung von HO-Radikalen zu Wasser und Sauerstoffoxyd als Zwischenprodukt, wobei die HO-Radikale im Reaktionsbereich des Katalysators ihre stark bakterizide Wirkung entfalten und die Luft desinfizieren.

Der Reinluftstrom verläßt den Katalysator, wobei das Wasserstoffperoxid zu Wasser und Sauerstoffoxyd umgesetzt ist.

**Patentansprüche**

1. Verfahren zur Desinfektion von Luft mit folgenden Arbeitsschritten:

   **a)** Anreichern der zu desinfizierenden Luft mit Wasserstoffperoxid;

   **b)** katalytische Zersetzung des Wasserstoffperoxid-Luftgemisches, um aus dem Wasserstoffperoxid HO-Radikale auszulösen;

   **c)** Einwirken der erzeugten HO-Radikale innerhalb des Katalysators auf das Wasserstoffperoxid-Luftgemisch, sodaß diese einerseits ihre bakterizide Wirkung auf den Luftstrom ausüben und sie andererseits unter Bildung neuer HO-Radikale in Form von Kettenreaktionen wiederholt mit dem Wasserstoffperoxid-Luftgemisch reagieren;

2. Vorrichtung zur Durchführung des Verfahrens nach nach Anspruch 1, dadurch gekennzeichnet, daß der Katalysator Platinmetalle oder Metalloxide als Wirkstoff aufweist.

3. Vorrichtung zur Durchführung des Verfahrens nach nach Anspruch 1, dadurch gekennzeichnet, daß der Katalysator als heterogener Katalysator ausgebildet ist und Stoffe mit rauher Oberfläche als Wirkstoff aufweist.

**Europäisches Patentamt**

## EUROPÄISCHER RECHERCHENBERICHT

Nummer der Anmeldung

EP 99 10 5622

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| Y | DATABASE WPI<br>Section Ch, Week 199230<br>Derwent Publications Ltd., London, GB;<br>Class D22, AN 1992-246373<br>XP002122555<br>& JP 04 166207 A (MITSUBISHI HEAVY IND CO LTD), 12. Juni 1992 (1992-06-12)<br>* Zusammenfassung *<br>--- | 1-3 | A61L9/00<br>A61L9/04 |
| P,Y | DE 197 36 336 A (HAGER HERBERT DIPL ING)<br>25. Februar 1999 (1999-02-25)<br>* Ansprüche *<br>--- | 1-3 | |
| A | EP 0 633 065 A (MANNESMANN AG)<br>11. Januar 1995 (1995-01-11)<br>* Ansprüche *<br>--- | 1-3 | |
| A | DATABASE WPI<br>Section Ch, Week 197939<br>Derwent Publications Ltd., London, GB;<br>Class E36, AN 1979-70881B<br>XP002122556<br>& JP 54 106094 A (HORIMOTO KOSAKUSHO),<br>20. August 1979 (1979-08-20)<br>* Zusammenfassung *<br>--- | 1-3 | |
| A | DATABASE WPI<br>Section Ch, Week 197912<br>Derwent Publications Ltd., London, GB;<br>Class D22, AN 1979-23127B<br>XP002122557<br>& JP 54 020956 A (MONMA K),<br>16. Februar 1979 (1979-02-16)<br>* Zusammenfassung *<br>----- | 1-3 | |

**RECHERCHIERTE SACHGEBIETE (Int.Cl.7)**

A61L

Der vorliegende Recherchenbericht wurde für alle Patentansprüche erstellt

| Recherchenort | Abschlußdatum der Recherche | Prüfer |
|---|---|---|
| DEN HAAG | 12. November 1999 | ESPINOSA, M |

EP 0 970 706 A1

**ANHANG ZUM EUROPÄISCHEN RECHERCHENBERICHT**
**ÜBER DIE EUROPÄISCHE PATENTANMELDUNG NR.**   EP 99 10 5622

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

12-11-1999

| Im Recherchenbericht angeführtes Patentdokument | Datum der Veröffentlichung | Mitglied(er) der Patentfamilie | Datum der Veröffentlichung |
|---|---|---|---|
| JP 4166207 A | 12-06-1992 | KEINE | |
| DE 19736336 A | 25-02-1999 | KEINE | |
| EP 0633065 A | 11-01-1995 | DE 4423329 A<br>AT 162112 T<br>DE 59405001 D<br>DK 633065 T<br>JP 7145723 A<br>US 5567392 A | 26-01-1995<br>15-01-1995<br>19-02-1998<br>09-03-1998<br>06-06-1995<br>22-10-1996 |
| JP 54106094 A | 20-08-1979 | KEINE | |
| JP 54020956 A | 16-02-1979 | KEINE | |

EPO FORM P0461

Für nähere Einzelheiten zu diesem Anhang : siehe Amtsblatt des Europäischen Patentamts, Nr. 12/82

4